# FASCICULE DE BREVET EUROPEEN

(11) **EP 2 644 981 B1**
(45) Date de publication et mention de la délivrance du brevet: **17.06.2015**
(21) Numéro de dépôt: 13158181.1
(22) Date de dépôt: 07.03.2013
(51) Int. Cl.: F21V 9/10, F21V 14/08, H05B 33/08, F21Y 101/02, F21W 131/205

(54) **Dispositif d'éclairage à LED blanche, appareil d'éclairage**
Weiße LED-Beleuchtungsvorrichtung, und Beleuchtungsapparat
White LED lighting device, lighting apparatus

(30) Priorité: 27.03.2012 FR 1252735
(43) Date de publication de la demande: 02.10.2013
(73) Titulaire: Maquet SAS, 45160 Ardon (FR)
(72) Inventeur: Jousse, Robin, 45380 LA CHAPELLE SAINT MESMIN (FR); Valteau, Cécilia, 45240 LIGNY LE RIBAULT (FR); Comte, Lionel, 45380 LA CHAPELLE SAINT MESMIN (FR)
(74) Mandataire: Prugneau, Philippe

(56) Documents cités:
- EP-A1- 1 462 711
- WO-A1-2008/087404
- DE-A1-102006 040 393
- GB-A- 2 430 248
- GB-A- 2 472 694

## Description

### Domaine technique

L'invention concerne un dispositif d'éclairage à LED comprenant une LED émettant une lumière blanche et un moyen de filtrage optique apte à filtrer ladite lumière blanche émise par la LED.

L'invention concerne aussi un appareil d'éclairage pour bloc opératoire comprenant un tel dispositif d'éclairage.

### Technique antérieure

Plus particulièrement, l'invention s'applique à un dispositif d'éclairage principalement destiné à être utilisé en milieu médical, en particulier en bloc opératoire. Un tel dispositif d'éclairage doit permettre à un chirurgien d'opérer dans de bonnes conditions, et notamment de distinguer correctement les différents types de tissus biologiques. Pour ce faire, le dispositif d'éclairage doit se conformer à certaines normes et produire une lumière globalement blanche qui présente un indice de rendu des couleurs (IRC) compris entre 85 et 100. En outre, la température de couleur de la lumière produite par le dispositif d'éclairage doit être comprise entre 3000 K (teintes chaudes) et 6700 K (teintes froides), selon la norme IEC 60601-2-41, pour permettre au chirurgien de distinguer sans effort de petites différences de couleur.

Par « température de couleur » d'une lumière, on entend la température de couleur équivalente évaluée, comme cela est bien connu, tout comme les coordonnées chromatiques (x,y), à partir du spectre de la lumière dans un diagramme chromatique de référence de la Commission Internationale de l'Eclairage.

On distinguera le flux lumineux d'une source de lumière, qui est la puissance lumineuse émise exprimée en lumen, et l'éclairement visuel d'un dispositif d'éclairage dans un champ d'éclairage, qui est la quantité de flux lumineux éclairant ce champ d'éclairage exprimée en lux, c'est-à-dire en lumen/m²_{.}

Selon l'opération à mener, le chirurgien peut avoir des besoins en éclairage très variés qui peuvent en outre évoluer en cours d'opération. Pour cela, il est souhaitable que le chirurgien puisse modifier les caractéristiques spectrales de la lumière produite par le dispositif d'éclairage, comme la température de couleur ou les coordonnées chromatiques, de sorte à obtenir différentes teintes de blanc adaptées à l'opération.

Actuellement, il existe différents types de dispositif d'éclairage répondant en partie aux exigences d'éclairage en milieu médical qui mélangent des LEDs blanches et de couleur pour obtenir une lumière d'éclairage blanche de température de couleur souhaitée, mais aucune ne propose de dispositif d'éclairage à caractéristique spectrale modifiable.

On connaît par exemple du document de brevet DE 102006040393 un dispositif d'éclairage à LEDs qui produit une lumière d'éclairage blanche à partir de LEDs émettant une lumière blanche froide, blanche chaude, rouge et verte. Ce dispositif utilise notamment un filtre optique placé directement sur une LED émettant une lumière blanche froide pour obtenir une lumière blanche chaude. Cependant, la température de couleur de la lumière d'éclairage émise par le dispositif d'éclairage n'est pas modifiable. En outre, il est connu que les filtres réduisent le flux lumineux de la lumière d'éclairage, ce qui n'est pas souhaitable.

On connaît aussi du document de brevet WO 2008/087404 un dispositif d'éclairage qui produit une lumière d'éclairage blanche à partir de LEDs blanches et rouges. En utilisant un filtre optique placé devant les LEDs blanches, ce dispositif d'éclairage permet d'obtenir une température de couleur de lumière d'éclairage souhaitée. Toutefois, ce dispositif d'éclairage ne permet pas au chirurgien de modifier la température de couleur de la lumière d'éclairage.

Un inconvénient des deux dispositifs précédents réside dans le fait que, lorsqu'un obstacle masque une partie du flux lumineux (lorsque le chirurgien se penche, par exemple), l'équilibre entre les contributions des différentes LEDs colorées est rompu, ce qui modifie la température de couleur de la lumière produite par le dispositif d'éclairage, produisant un effet d'irisation, et forme des ombres colorées dans le champ d'éclairage.

### Exposé de l'invention

Le but de l'invention est de remédier à tous ces inconvénients en proposant un dispositif d'éclairage à LED produisant dans un champ d'éclairage une lumière d'éclairage blanche dont les caractéristiques spectrales, comme par exemple la température de couleur, sont réglables par le chirurgien sans baisse de l'éclairement visuel dans le champ d'éclairage.

A cet effet, l'invention a pour objet un dispositif d'éclairage à LED comprenant une LED émettant une lumière blanche et un moyen de filtrage optique apte à filtrer ladite lumière blanche émise par la LED, **caractérisé en ce que** ledit moyen de filtrage optique comprend au moins deux filtres optiques ayant des coefficients de transmission différents et qui sont positionnables pour filtrer individuellement ladite lumière émise par la LED et en ce que le dispositif d'éclairage comprend une unité d'alimentation apte à fournir des intensités de courant différentes à ladite LED selon que l'un ou l'autre des filtres optiques est positionné pour filtrer ladite lumière de la LED, de sorte à modifier la température de couleur de la lumière émise par la LED.

Avec un tel agencement du dispositif d'éclairage selon l'invention, les caractéristiques spectrales de l'éclairement dans le champ d'éclairage, notamment la température de couleur ou les coordonnées chromatiques donc la teinte de blanc, sont facilement réglables par simple changement de filtre optique sans modification de l'éclairement visuel dans le champ d'éclairage, puisque le flux lumineux de la LED est conservé grâce à l'alimentation variable de la LED.

Un autre avantage du dispositif d'éclairage selon l'invention est que, grâce à l'utilisation de LEDs blanches uniquement, la température de couleur obtenue n'est pas modifiée en cas de présence d'obstacle dans le flux lumineux.

Par exemple, le dispositif d'éclairage selon l'invention pourra avantageusement comprendre au moins un filtre optique apte à atténuer une composante rouge de la lumière émise par la LED de sorte à atténuer l'éclairement énergétique de la LED, c'est-à-dire baisser le radiant énergétique. Un tel filtre optique, encore appelé « filtre froid » permet de bloquer les plus grandes longueurs d'ondes tout en restant neutre au sens de la perception des couleurs par l'oeil humain dans le domaine visible, c'est-à-dire qu'un tel filtre ne modifie ni la température de couleur, ni l'indice de rendu des couleurs de la lumière produite par le dispositif d'éclairage.

Avec le dispositif d'éclairage selon l'invention, on peut donc réduire le radiant énergétique de la source de lumière qui est à la fois une source d'assèchement pour le patient et gênant pour le personnel médical. On rappelle que le radiant énergétique est défini classiquement comme le rapport entre l'éclairement énergétique exprimé en Watt/m² et l'éclairement exprimé en lux. L'éclairement énergétique intègre tous les rayonnements entre 300 nm et 2500 nm. Pour comparaison, l'éclairement visuel intègre l'éclairement entre 360 nm et 780 nm pondéré par la visibilité de l'oeil qui est quasiment nulle au-delà de 700 nm. Un filtre optique « froid » tel que décrit ci-dessus permet donc de baisser l'éclairement énergétique sans modifier l'éclairement visuel et sans affecter ni la température de couleur, ni les autres propriétés colorimétriques.

Avantageusement, le dispositif d'éclairage selon l'invention pourra comprendre au moins un filtre optique apte à atténuer une composante bleue de la lumière émise par la LED à une longueur d'onde comprise entre 400 nanomètres et 480 nanomètres. Un tel filtre optique, appelé ici filtre « bleu », permet de fournir un éclairement du champ d'éclairage équilibré sur l'ensemble des longueurs d'onde visibles offrant une très bonne perception des couleurs au chirurgien, et limite les risques photobiologiques.

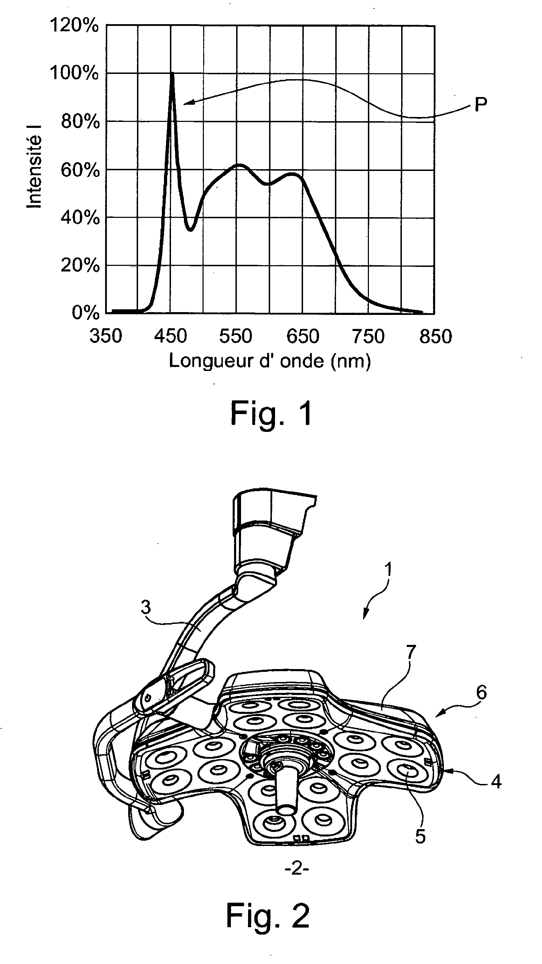

En effet, il est connu que les diodes électroluminescentes (ou LED pour Light Emitting Diode), en particulier les LEDs blanches, possèdent dans leur spectre d'émission une forte proportion de composante bleue, désignée communément par l'expression « pic bleu ». Un tel déséquilibre des composantes de la lumière blanche émise par les LEDs affecte la perception des couleurs par le chirurgien. En outre, il est connu que des composantes bleues en excès au sens de la norme EN62471 augmentent le risque photobiologique. La Figure 1 représente un exemple de spectre d'émission d'une LED blanche, c'est-à-dire l'intensité I relative émise par la LED en fonction de la longueur d'onde de la lumière émise par la LED. L'exemple de la Figure1 illustre bien, indiqué par la flèche P, la présence dans le spectre d'émission d'un pic bleu de courtes longueurs d'onde comprises entre environ 400 nm et 480 nm et centré aux alentours de 450 nm.

Un filtre optique « bleu » tel que défini ci-dessus permet d'atténuer la lumière dans une plage de longueurs d'onde où le risque photobiologique bleu, défini par exemple dans la norme EN 62471 : 2008, est le plus élevé. En outre, un tel filtre optique « bleu » permet avantageusement de diminuer la température de couleur de la lumière produite par le dispositif d'éclairage, ce qui offre au chirurgien une possibilité de faire varier simplement et facilement la température de couleur de l'éclairage pour l'adapter à ses besoins.

Un dispositif d'éclairage selon l'invention peut présenter avantageusement les particularités suivantes:
- le dispositif d'éclairage comprend une pluralité de filtres optiques consécutifs ayant des coefficients de transmission progressifs d'atténuation de la composante bleue;
- l'écart d'atténuation entre deux filtres optiques consécutifs est d'au moins 15 %;
- le dispositif d'éclairage comprend une pluralité de LEDs identiques émettant chacune une lumière blanche ;
- à chaque LED est associée un jeu de filtres optiques de coefficients de transmission différents, les jeux de filtres optiques étant identiques d'une LED à l'autre.
- lesdits filtres optiques sont disposés sur un disque rotatif ;
- lesdits filtres sont disposés à la périphérie dudit disque rotatif.

L'invention s'étend à un appareil d'éclairage pour bloc opératoire comprenant au moins un tel dispositif d'éclairage.

### Description sommaire des dessins

La présente invention sera mieux comprise et d'autres avantages apparaîtront à la lecture de la description détaillée d'un mode de réalisation pris à titre d'exemple nullement limitatif et illustré par les dessins annexés dans lesquels :
- la figure 1 est un diagramme montrant un spectre d'émission d'une LED blanche ;
- la figure 2 est une vue schématique en perspective d'un appareil d'éclairage comprenant quatre dispositifs d'éclairage selon l'invention;
- la figure 3 est une vue schématique en perspective de la partie arrière de l'un des dispositifs d'éclairage de la figure 2 ;
- la figure 4 est une vue schématique en coupe selon l'axe IV-IV de la figure 3 d'une partie du dispositif d'éclairage de la figure 2 ;
- la figure 5 représente des coefficients de transmission de trois filtres optiques d'un dispositif d'éclairage selon l'invention.

### Description des modes de réalisation

Sur la figure 2, on a illustré un appareil d'éclairage 1 éclairant un champ d'éclairage opératoire 2, ici un champ d'intervention d'un chirurgien sur un patient.

L'appareil d'éclairage 1 est ici du type suspendu au plafond de la salle opératoire de façon connue en soi, et comprend un bras de suspension 3 articulé portant une coupole 4 d'éclairage.

Comme on peut le voir sur la figure 2, la coupole 4 a ici une forme de croix dont chaque branche comprend un dispositif d'éclairage 6 selon l'invention inséré dans un boîtier 7 de coupole 4. Dans le cas d'exemple, les dispositifs d'éclairage 6 sont sensiblement identiques et comportent chacun quatre sorties d'éclairage 5 aussi sensiblement identiques.

Avantageusement, les dispositifs d'éclairage 6 comportent des LEDs 8 agencées pour émettre une lumière blanche et, de préférence, chaque sortie d'éclairage 5 comprend une seule LED 8 blanche. Dans le cas d'exemple, la LED 8 émet une lumière blanche d'une température de couleur de 5000 K.

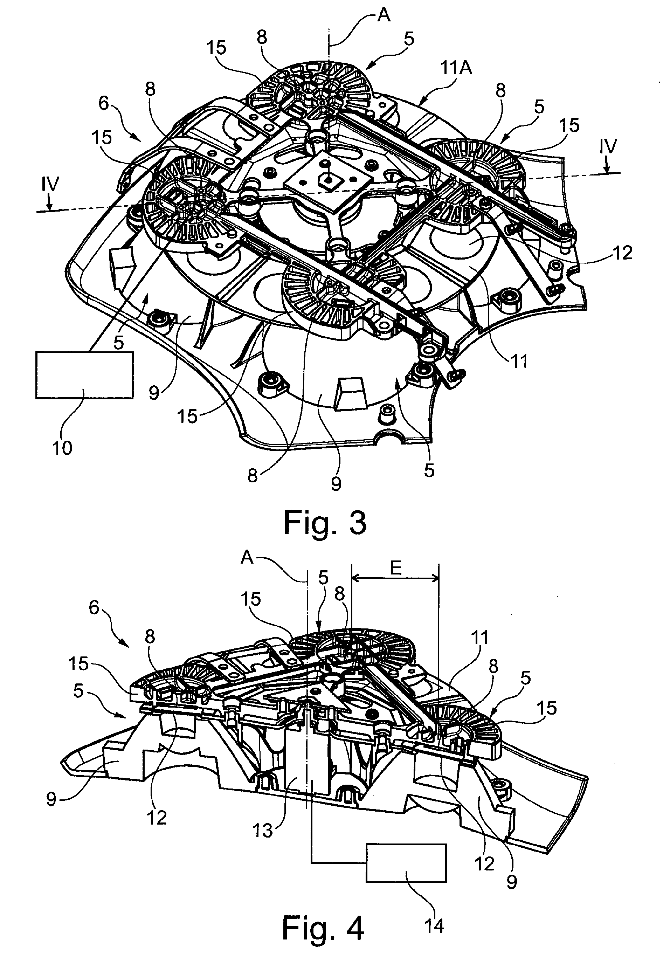

On a représenté schématiquement sur la figure 3 une vue agrandie d'un dispositif d'éclairage 6 selon l'invention sans le boîtier 7 pour mieux faire apparaître ses éléments optiques.

Comme on peut le voir sur la figure 3, chaque sortie d'éclairage 5 comprend donc la LED 8 blanche, devant laquelle est disposé un collimateur 9 connu en soi pour diriger le flux lumineux de la LED 8 vers le champ d'éclairage 2 et, interposé entre le collimateur 9 et la LED 8, un moyen de filtrage optique 12 de la lumière émise par la LED 8. Un radiateur 15 est de préférence disposé adjacent à la LED 8 pour dissiper la chaleur produite par la LED 8.

Sur la figure 4, on a représenté le dispositif d'éclairage 6 en coupe transversale selon l'axe IV-IV de la figure 3 pour mieux faire apparaître que chaque filtre optique 12 est disposé entre une LED 8 et un collimateur 9, cette configuration permettant d'utiliser des filtres optiques 12 de taille réduite.

Avantageusement, le moyen de filtrage optique comprend, pour chaque LED 8, plusieurs filtres optiques 12, ici trois filtres optiques 12, consécutifs ayant des coefficients de transmission différents et de préférence progressifs, et qui sont positionnables pour filtrer individuellement la lumière émise par chaque LED 8 de sorte à obtenir différentes teintes de blanc, c'est-à-dire une température de couleur variable ou des coordonnées chromatiques variables.

Plus précisément, le dispositif d'éclairage 6 comprend un support 11 des filtres optiques 12 monté pivotant autour d'un axe A de sorte que les filtres optiques 12 et les LEDs 8 sont mobiles les uns par rapport autres. Le support 11 se présente ici sous la forme d'un disque, les filtres optiques 12 étant disposés sur la périphérie 11A du disque 11 et alignés selon un cercle centré sur l'axe A, et les LEDs 8 étant disposées aux quatre coins d'un carré inscrit dans le cercle formé par les filtres optiques 12 de sorte que, lorsque le disque 11 pivote autour de l'axe A, les filtres optiques 12 occupent des positions successives dans lesquelles ils sont alignés axialement avec les LEDs 8 pour être traversés par la lumière blanche émise par les LEDs 8. Ainsi, on comprend bien que, pour chaque position du disque 11 autour de l'axe A, chaque LED 8 est alignée axialement avec un filtre optique 12 qui est par conséquent traversé par la lumière blanche émise par cette LED 8.

En outre, les LEDs 8 sont alimentées en courant par une unité d'alimentation 10 électrique (représentée ici schématiquement uniquement pour une sortie d'éclairage 5), agencée pour alimenter chaque LED 8 en courant d'intensités différentes et variables selon que l'un ou l'autre des filtres optiques 12 est positionné devant la LED 8 pour en filtrer la lumière. L'unité d'alimentation 10 peut se présenter sous la forme d'une alimentation unique pour toutes les LEDs 8 du dispositif d'éclairage 6 ou sous la forme de plusieurs alimentations respectives associées à chaque LED 8.

Avantageusement, à chaque fois que le disque 11 pivote autour de l'axe A pour aligner les LEDs 8 avec un type de filtre optique 12, l'unité d'alimentation ajuste l'intensité des courants traversant les LEDs 8 de façon à conserver sensiblement constant le flux lumineux sortant du dispositif d'éclairage 6, c'est-à-dire un éclairement visuel dans le champ d'éclairage sensiblement constant, et ce quelque soit le type de filtre placé devant les LEDs 8. Par sensiblement constant, on entend que le flux lumineux est identique à 5% près à chaque changement de filtre optique 12.

De préférence, pour chaque position du disque 11 autour de l'axe A, les quatre LEDs 8 sont alignées avec des filtres optiques 12 du même type, de sorte que le dispositif d'éclairage 6 a un flux lumineux homogène. Pour cela, on peut prévoir que l'entraxe entre deux LEDs 8 successives noté E sur la figure 4 est sensiblement égal à l'entraxe entre deux filtres optiques 12 de même type appartenant à deux jeux de filtres successifs.

Les filtres optiques 12 sont spécifiés de préférence de façon à ce que les températures de couleur minimale et maximale obtenues après atténuation du pic bleu se situent dans l'intervalle autorisé par la norme IEC 60601-2-41. On choisira en particulier des températures de couleur couramment rencontrées dans les blocs opératoires.

Dans le cas d'exemple, le disque 11 comporte au total douze filtres optiques 12 répartis en quatre jeux identiques associés chacun à une LED et comprenant donc chacun trois filtres optiques 12 de types différents et progressifs permettant d'obtenir trois températures de couleur différentes et progressives.

Par « progressif», on entend que les filtres optiques 12 sont choisis de sorte à établir un écart de transmission entre deux types de filtres optiques successifs d'un même jeu de filtres optiques 12, de préférence, cet écart de transmission est d'au moins 15%. Dans le cas d'exemple, au sein d'un jeu de trois filtres optiques associé à une LED 8, le premier filtre optique 12 a ainsi un premier coefficient de transmission qui ne modifie pas la température de couleur et permet d'obtenir une température de couleur maximale, ici 5000 K, le deuxième filtre optique 12 a un deuxième coefficient de transmission qui permet d'obtenir une température de couleur intermédiaire, ici 4500 K, et le troisième filtre optique 12 a un troisième coefficient de transmission qui permet d'obtenir une température de couleur minimale, ici 3900 K.

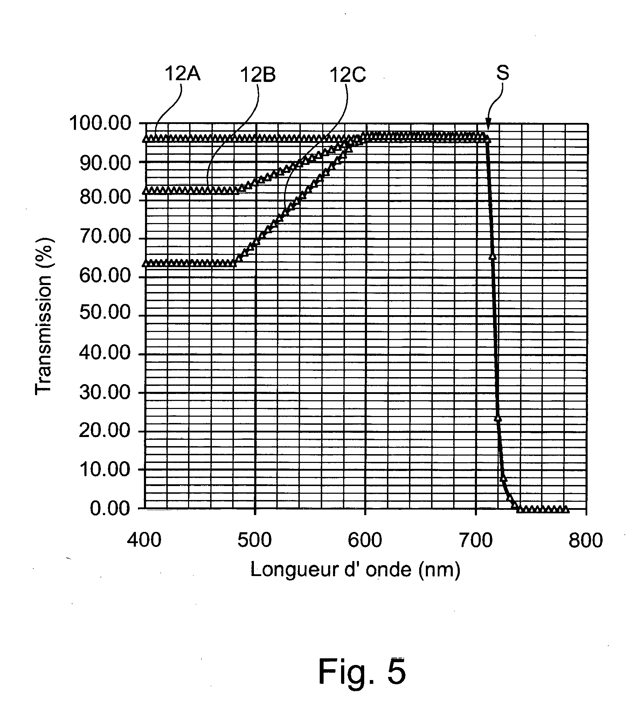

La figure 5 représente, en fonction de la longueur d'onde, un exemple de coefficients de transmission 12A, 12B, 12C de trois filtres optiques progressifs d'un jeu de filtres optiques 12. La figure 5 est donnée à titre d'exemple pour un angle d'incidence de la lumière sur les filtres de 30°. Pour un autre angle d'incidence, les coefficients de transmission varient légèrement en fonction de la longueur d'onde.

Comme on peut le voir sur la figure 5, les trois filtres optiques 12A, 12B, 12C sont aptes à absorber une composante rouge de la lumière émise par la LED 8 pour diminuer le radiant énergétique sans modifier la température de couleur, ni le rendu des couleurs de la lumière émise par la LED 8. La baisse du radiant énergétique est de l'ordre de 0,4 à 0,5 mW.m⁻².lux⁻¹. Sur l'exemple de la figure 5, les coefficients de transmission des trois filtres optiques 12A, 12B, 12C présentent un seuil au-delà duquel le coefficient de transmission chute pour atteindre 2% pour des longueurs d'onde supérieures à 740 nm. Ce seuil indiqué par S sur la figure 5, se situe ici à une longueur d'onde d'environ 700 nanomètres, mais il pourrait se situer entre 670 nm et 750 nm, et sera adapté notamment selon le type de LED 8 utilisé et l'angle d'incidence de la lumière émise par la LED 8 sur le filtre.

On voit également sur la figure 5 que le premier filtre optique a un coefficient de transmission de 96% +/- 2% pour les longueurs d'onde inférieures à 700 nm. Ce premier filtre optique 12A permet donc de conserver la température de couleur de la lumière émise par la LED 8 tout en limitant la production de chaleur.

Enfin, la figure 5 montre que les deuxième et troisième filtres optiques 12B, 12C atténuent en plus une composante bleue de la lumière blanche émise par la LED 8 correspondant au pic bleu du spectre d'émission de la figure 1, c'est-à-dire de longueur d'onde aux alentours de 450 nm, et de préférence en atténuant des composantes bleues de longueurs d'ondes comprises entre environ 400 nm et 480 nm.

Plus précisément, le deuxième filtre optique 12B a un coefficient de transmission égal à 83% +/- 2% pour des longueurs d'onde comprises entre 400 nm et 480 nm, puis un coefficient de transmission qui augmente avec la longueur d'onde de 83% jusqu'à 97% +/- 2% pour des longueurs d'onde comprises entre 480 nm et 620 nm. Le troisième filtre 12C a un coefficient de transmission égal à 64% +/- 2% pour des longueurs d'onde comprises entre 400 nm et 480 nm, puis, un coefficient de transmission qui augmente avec la longueur d'onde de 64% jusqu'à 97% +/- 2% pour des longueurs d'onde comprises entre 480 nm et 620 nm. Ces deuxième et troisième filtres optiques 12B, 12C permettent donc de modifier la température de couleur de la lumière émise par la LED 8 en diminuant chacun la température de couleur de 600 K, tout en limitant la production de chaleur. Ainsi, le moyen de filtrage optique 12 permet de modifier la température de couleur de la lumière émise par la LED 8.

Selon l'application, les filtres optiques 12 peuvent être en verre ou en matière plastique. De préférence, les filtres optiques 12 sont fabriqués par dépôt de couche mince sous vide ou par voie sol-gel, sur un substrat transparent.

Le disque 11 portant les filtres optiques 12 pourra avantageusement être entraîné en rotation par un moteur 13 commandé par une unité de commande 14 représentés sur la figure 4, de façon à ce que le chirurgien puisse changer simplement et rapidement de filtres optiques 12 par actionnement de l'unité de commande 14.

De préférence, les LEDs 8 des sorties d'éclairage 5 sont sensiblement identiques les unes aux autres pour s'affranchir de toute différence de flux lumineux et/ou de spectre dans le domaine visible d'une sortie d'éclairage 5 à l'autre. En particulier, on choisira de préférence des LEDs 8 provenant du même fournisseur, ayant par exemple un même type de composé à base de phosphore, un même boîtier, des mêmes puces électroniques et nécessitant un même type d'alimentation. On choisira des LEDs 8 blanches ayant un indice de rendu des couleurs élevé, compris entre 85 et 100, de préférence compris entre 90 et 100, voire compris entre 95 et 100 et une température de couleur comprise entre 3000 K et 6700 K pour répondre aux normes en vigueur pour l'éclairage en milieu médical.

Il va de soi que la présente invention ne saurait être limitée à la description qui précède d'un de ses modes de réalisation, susceptibles de subir quelques modifications sans pour autant sortir du cadre de l'invention.

Par exemple, on pourra bien entendu disposer un nombre différent de filtres optiques 12 associés à chaque LED dans le dispositif d'éclairage 6. On pourra aussi prévoir différents nombres de LEDs 8 et de sorties d'éclairage 5 dans le dispositif d'éclairage 6. Enfin, on pourra prévoir un nombre différent de dispositifs d'éclairage 6 pour chaque coupole 4. La forme en croix de la coupole 4 est donnée uniquement à titre d'exemple.

On pourra par exemple utiliser une LED émettant une lumière blanche à une température de couleur basse, par exemple 3000 K. Dans ce cas, les filtres optiques 12 seront choisis de sorte à pouvoir augmenter la température de couleur de la lumière, par exemple en atténuant des composantes rouges de la lumière entre 600 nm et 700 nm.

On pourra aussi prévoir d'utiliser d'autres types de filtres optiques, par exemple atténuant des composantes ultraviolettes de la lumière.

## Revendications

1. Dispositif d'éclairage (6) à LED comprenant une LED (8) émettant une lumière blanche et un moyen de filtrage optique (12) apte à filtrer ladite lumière blanche émise par la LED (8), **caractérisé en ce que** ledit moyen de filtrage optique comprend au moins deux filtres optiques (12) ayant des coefficients de transmission différents et qui sont positionnables pour filtrer individuellement ladite lumière émise par la LED (8) et **en ce que** le dispositif d'éclairage (6) comprend une unité d'alimentation (10) apte à fournir des intensités de courant différentes à ladite LED (8) selon que l'un ou l'autre des filtres optiques (12) est positionné pour filtrer ladite lumière de la LED (8), de sorte à modifier la température de couleur de la lumière émise par la LED (8).

2. Dispositif d'éclairage (6) selon la revendication 1, dans lequel au moins un filtre optique (12) est apte à atténuer une composante rouge de la lumière émise par la LED (8).

3. Dispositif d'éclairage (6) selon la revendication 1 ou 2, dans lequel au moins un filtre optique (12) est apte à atténuer une composante bleue de la lumière émise par la LED (8) à une longueur d'onde comprise entre 400 nanomètres et 480 nanomètres.

4. Dispositif d'éclairage (6) selon la revendication 3, comprenant une pluralité de filtres optiques (12) consécutifs ayant des coefficients de transmission progressifs d'atténuation de la composante bleue.

5. Dispositif d'éclairage (6) selon la revendication 4, dans lequel l'écart d'atténuation entre deux filtres optiques (12) consécutifs est d'au moins 15 %.

6. Dispositif d'éclairage (6) selon l'une quelconque des revendications précédentes, comprenant une pluralité de LEDs (8) identiques émettant chacune une lumière blanche.

7. Dispositif d'éclairage (6) selon la revendication 6, dans lequel à chaque LED (8) est associée un jeu de filtres optiques (12) de coefficients de transmission différents, les jeux de filtres optiques (12) étant identiques d'une LED (8) à l'autre.

8. Dispositif d'éclairage (6) selon l'une quelconque des revendications 1 à 7, dans lequel lesdits filtres optiques (12) sont disposés sur un disque (11) rotatif.

9. Dispositif d'éclairage (6) selon la revendication 8, dans lequel lesdits filtres (12) sont disposés à la périphérie (11 A) dudit disque (11) rotatif.

10. Appareil d'éclairage (1) pour bloc opératoire **caractérisé en ce qu'il** comprend au moins un dispositif d'éclairage (6) selon au moins l'une des revendications 1 à 9.

## Patentansprüche

1. LED-Beleuchtungsvorrichtung (6), die eine LED (8), die ein weißes Licht emittiert, und ein optisches Filterungsmittel (12) umfasst, das geeignet ist, das durch die LED (8) emittierte weiße Licht zu filtern, **dadurch gekennzeichnet, dass** das optische Filterungsmittel mindestens zwei optische Filter (12) umfasst, die unterschiedliche Transmissionskoeffizienten aufweisen und die positioniert werden können, um das durch die LED (8) emittierte Licht einzeln zu filtern, und dadurch, dass die Beleuchtungsvorrichtung (6) eine Versorgungseinheit (10) umfasst, die geeignet ist, der LED (8), je nachdem, ob das eine oder das andere der optischen Filter (12) positioniert ist, um das Licht der LED (8) zu filtern, unterschiedliche Stromstärken bereitzustellen, derart, dass die Farbtemperatur des durch die LED (8) emittierten Lichts geändert wird.

2. Beleuchtungsvorrichtung (6) nach Anspruch 1, wobei mindestens ein optisches Filter (12) geeignet ist, eine rote Komponente des durch die LED (8) emittierten Lichts zu dämpfen.

3. Beleuchtungsvorrichtung (6) nach Anspruch 1 oder 2, wobei mindestens ein optisches Filter (12) geeignet ist, eine blaue Komponente des durch die LED (8) emittierten Lichts bei einer Wellenlänge zu filtern, die zwischen 400 Nanometern und 480 Nanometern enthalten ist.

4. Beleuchtungsvorrichtung (6) nach Anspruch 3, die mehrere aufeinanderfolgende optische Filter (12) umfasst, die progressive Transmissionskoeffizienten zur Dämpfung der blauen Komponente aufweisen.

5. Beleuchtungsvorrichtung (6) nach Anspruch 4, wobei der Dämpfungsunterschied zwischen zwei aufeinanderfolgenden optischen Filtern (12) mindestens 15 % beträgt.

6. Beleuchtungsvorrichtung (6) nach einem der vorhergehenden Ansprüche, die mehrere identische LEDs (8) umfasst, die jeweils ein weißes Licht emittieren.

7. Beleuchtungsvorrichtung (6) nach Anspruch 6, wobei jede LED (8) einer Gruppe optischer Filter (12) mit unterschiedlichen Transmissionskoeffizienten zugehörig ist, wobei die Gruppen von optischen Filtern (12) von einer LED (8) zur anderen identisch sind.

8. Beleuchtungsvorrichtung (6) nach einem der Ansprüche 1 bis 7, wobei die optischen Filter (12) auf einer Drehscheibe (11) angeordnet sind.

9. Beleuchtungsvorrichtung (6) nach Anspruch 8, wobei die Filter (12) am Umfang (11A) der Drehscheibe (11) angeordnet sind.

10. Beleuchtungsapparat (1) für einen Operationstrakt, **dadurch gekennzeichnet, dass** er mindestens eine Beleuchtungsvorrichtung (6) nach mindestens einem der Ansprüche 1 bis 9 umfasst.

## Claims

1. A LED lighting device (6) having a LED (8) emitting white light and optical filter means (12) suitable for filtering said white light emitted by the LED (8), the device being **characterized in that** said optical filter means comprise at least two optical filters (12) that have different transmission coefficients and that are positionable to filter said light emitted by the LED (8) individually, and **in that** the lighting device (6) comprises a power supply unit (10) suitable for delivering different power supply currents to said LED (8) depending on whether one or the other of the optical filters (12) is positioned to filter said light from the LED (8), so as to modify the color temperature of the light emitted by the LED (8).

2. A lighting device (6) according to claim 1, wherein at least one optical filter (12) is suitable for attenuating a red component of the light emitted by the LED (8).

3. A lighting device (6) according to claim 1 or claim 2, wherein at least one optical filter (12) is suitable for attenuating a blue component of the light emitted by the LED (8) at a wavelength lying in the range 400 nm to 480 nm.

4. A lighting device (6) according to claim 3, comprising a plurality of consecutive optical filters (12) having progressive transmission coefficients for attenuating the blue component.

5. A lighting device (6) according to claim 4, wherein the attenuation difference between two consecutive optical filters (12) is at least 15%.

6. A lighting device (6) according to any preceding claim, comprising a plurality of identical LEDs (8), each emitting white light.

7. A lighting device (6) according to claim 6, wherein each LED (8) is associated with a set of optical filters (12) having different transmission coefficients, the sets of optical filters (12) being identical from one LED (8) to another.

8. A lighting device (6) according to any one of claims 1 to 7, wherein said optical filters (12) are arranged on a rotary disk (11).

9. A lighting device (6) according to claim 8, wherein said optical filters (12) are arranged at the periphery (11A) of said rotary disk (11).

10. A lighting appliance (1) for an operating theater, the appliance being **characterized in that** it comrpises at least one lighting device (6) according to at least any one of claims 1 to 9.
